# DEMANDE DE BREVET EUROPEEN

(11) **EP 2 112 132 A1**
(43) Date de publication de la demande: **28.10.2009**
(21) Numéro de dépôt: 08300190.9
(22) Date de dépôt: 22.04.2008
(51) Int. Cl.: C07C 45/00, C07C 45/50, C07C 45/51, C07C 47/115, C07C 49/323, C07C 1/22, C07C 5/05, C07C 41/56, C07C 43/303

(54) **Nouveaux aldehydes a structures norbornane, leur preparation et utilisation en parfumerie**

(71) Demandeur: V. Mane Fils, 06620 Bar sur Loup (FR)
(72) Inventeur: Mane, Jean, 06130, GRASSE (FR); Clinet, Isabel, 06270, VILLENEUVE-LOUBET (FR); Muratore, Agnes, 06560, Valbonne (FR); Clinet, Jean-Claude, 06270, VILLENEUVE-LOUBET (FR); Chanot, Jean-Jacques, 06530, SPERACEDES (FR)
(74) Mandataire: Cabinet Plasseraud

(57) **Abrégé**

L'invention concerne un aldéhyde à structure norbornane de formule I. L'invention concerne sa préparation et son utilisation en tant qu'agent ou composé odorant.

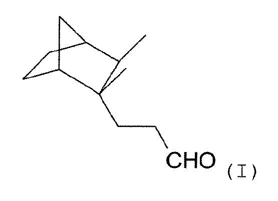

## Description

La présente invention concerne de nouveaux aldéhydes à structure norbornane présentant une fragrance particulière, notamment une odeur boisée, patchouli, avec des notes iris marquées. L'invention concerne donc également l'utilisation de ces aldéhydes en parfumerie.

Un autre objet de l'invention est la préparation de ces aldéhydes.

Les termes « fragrance », « fragrant », « odorant » sont utilisés ici de manière interchangeable pour tout composé organoleptique stimulant de façon plaisante l'odorat.

Le terme « composé organoleptique » est utilisé ici pour tout composé stimulant les sens de l'odorat et du goût, et étant donc perçu comme possédant une odeur et/ou un goût caractéristique.

Par le terme « masquer » ou « masquant » on entend réduire ou éliminer la perception d'une mauvaise odeur ou d'un mauvais goût générée par une ou plusieurs molécules entrant dans la composition d'un produit.

L'huile de bois de santal qui est obtenu par distillation vapeur à partir du bois et des racines de l'arbre de bois de santal présente une fragrance très agréable, sucrée-boisée avec des propriétés excellentes de fixation. A cause de son prix élevé, de sa disponibilité limitée et de l'importance de l'huile de santal en parfumerie, la chimie de l'odeur de santal a fait l'objet de nombreuses recherches. L'identification des deux principales molécules conférant son aspect olfactif à l'huile de bois de santal naturelle, à savoir l'α- et le β-santalol, a mis en évidence l'intérêt de la structure carbonée bicyclo[2.2.1]heptane, comme squelette de molécules présentant des fragrances intéressantes. Ainsi, le brevet US 4,229,600 a pour objet la préparation de dérivés à structure norbornane ou norbornène ayant l'odeur d'huile de bois de santal.

La demande de brevet FR 2 831 165 dont la Société Demanderesse est la titulaire décrit des dérivés de structure cis-2,3-dimethylbicyclo[2.2.1]heptane et plus particulièrement la cétone II (également appelée Présantone H^{®}), sous la forme d'un mélange d'isomères *endo* et *exo*. Cette cétone bicyclique et certains de ses dérivés (alcools, éthers, esters) présentent une large gamme de notes boisées puissantes et tenaces, d'un intérêt certain en parfumerie.

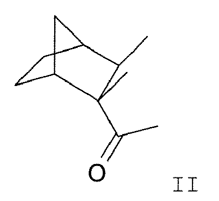

Par ailleurs, la cétone II est préparée avec de bons rendements à partir de substances bon marché, ce qui en fait un produit de départ intéressant pour l'élaboration de nouvelles fragrances. Ainsi, la Société Demanderesse a pu après des travaux de recherches approfondis mettre au point des voies de synthèses permettant l'obtention, avec un rendement satisfaisant, d'un aldéhyde de norbornane, non connu jusqu'à présent. Cet aldéhyde est particulièrement intéressant sur le plan olfactif.

Un premier objet de la présente invention est donc un dérivé de norbornane de formule I.

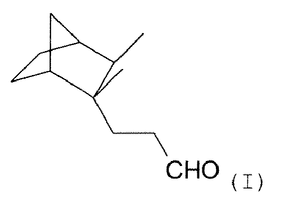

L'invention comprend tous les isomères de formule I, seuls ou en mélange. Sont notamment compris les mélanges d'isomères *exo* et *endo*, ainsi que les isomères I-*endo* et I-*exo* comme régioisomères purs.

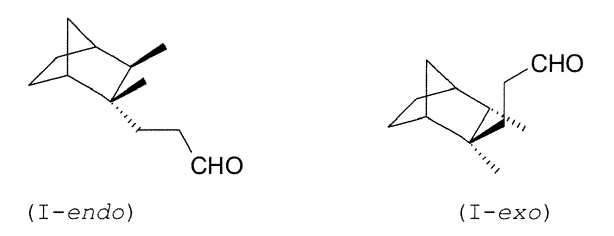

La présence dans la structure des composés I de centres d'asymétrie provoque l'existence, pour chacun d'entre eux, de formes énantiomériques. L'invention comprend les composés représentés par la formule générale I (*endo* et *exo*) sous forme de mélanges des énantiomères en proportions variables, en particulier les mélanges racémiques. L'invention comprend également les composés de formule I (*endo* et *exo*) sous forme d'un seul énantiomère. L'élaboration des mélanges d'énantiomères ou de formes pures est réalisée par des méthodes connues de l'homme de l'art en utilisant par exemple, comme produits de départ, des substances a structure *cis-2,3-*dimethylbicyclo[2.2.1]heptane, telles que la cétone II, optiquement enrichies ou optiquement pures.

La présente invention a également pour objet l'utilisation de l'aldéhyde I, sous forme d'un mélange des régioisomères ou sous des régioisomères purs, comme agent ou composé odorant, notamment en tant que fragrance ou agent masquant. Un panel d'évaluation, composé de plusieurs professionnels, a évalué qualitativement le composé de l'invention. L'aldéhyde de formule I, sous la forme d'un mélange d'isomères *endo* et *exo*, est très intéressant olfactivement. En effet, ce mélange présente des notes olfactives boisées, patchouli et irisées. Chacun des régioisomères *endo* et *exo* présente des aspects olfactifs différents et intéressants dans le domaine de la parfumerie. En effet, le régioisomère I-*endo* possède une fragrance puissante, boisée-ambrée avec des notes patchouli et iris très perceptibles. Le régioisomère I-*exo*, quant à lui, présente des notes vertes, terpéniques, rappelant les aiguilles de pin.

Du fait de ses qualités olfactives, l'aldéhyde I, sous forme d'un mélange des régioisomères ou sous forme des régioisomères purs, trouve un emploi très varié en parfumerie pour la préparation de compositions parfumantes, de bases ou concentrés parfumants, parfums ou eaux de toilette, ainsi que dans la préparation d'articles parfumés divers, tels que savons, bain moussant, gels douches ou de bain, lotions après rasage, shampooings ou autres produits capillaires notamment d'hygiène capillaire ou de soin capillaire, préparations cosmétiques, désodorisants corporels ou d'air ambiant, ou encore détergents, lessives ou adoucissants textiles ou produits d'entretien ou d'assainissement de l'air ambiant.

Dans ces applications, le composé de l'invention, sous forme d'un seul régioisomère ou d'un mélange de régioisomères, peut être utilisé seul ou, comme il est courant en parfumerie, en mélange avec d'autres ingrédients parfumants, des solvants ou des adjuvants d'usage courant en parfumerie et que l'homme de métier est à même de choisir en fonction de l'effet désiré et de la nature du produit à parfumer.

Les concentrations dans lesquelles l'aldéhyde I, sous forme d'un seul régioisomère ou d'un mélange de régioisomères, peut être utilisé pour obtenir les effets parfumants et/ou masquants désirés varient dans une gamme de valeur très étendue, de 0,001% à 99% en poids, de préférence de 0,001% à 20% en poids, très préférentiellement de 0,1% à 10% en poids, étant bien connu que ces valeurs dépendent de la nature de l'article à parfumer, de l'effet odorant recherché, et de la nature des autres ingrédients dans une composition donnée.

Un autre objet de l'invention est la préparation de l'aldéhyde I. L'aldéhyde de formule I peut être synthétisé à partir de la cétone de formule II.

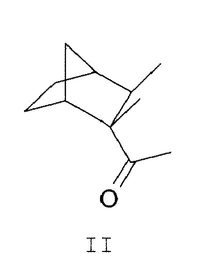

Un procédé de préparation d'un aldéhyde de formule I à partir de la cétone II par une synthèse multi-étapes est donc un autre objet de l'invention.

Dans un premier aspect ce procédé comprend :
1. une étape de transformation de la cétone II en le dérivé acétylénique III correspondant, 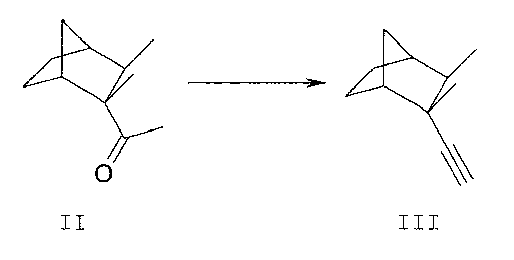
2. une étape transformation du dérivé III en l'oléfine IV correspondante, et 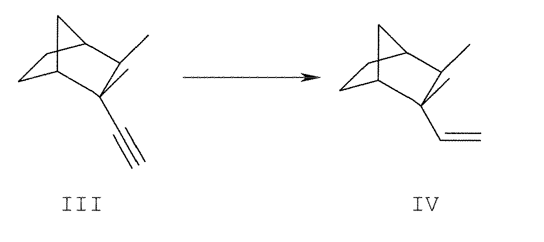
3. une étape d'hydroformylation du dérivé IV pour obtenir l'aldéhyde I.

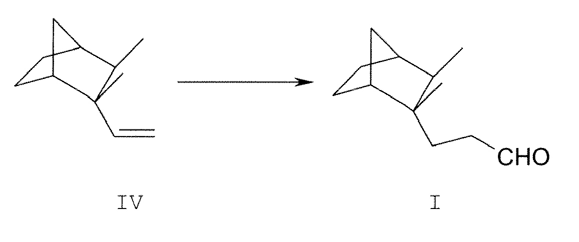

De préférence, la transformation de l'étape 1 est effectuée par une réaction de la cétone II avec 1) du lithium diisopropylamide / diéthylchlorophosphate et 2) du lithium diisopropylamide / H₂O selon Negishi et al, Organic Synthesis, Coll. Vol. VII, 63.

La seconde étape peut être effectuée par hydrogénation du dérivé III avec le catalyseur de Lindlar ou par une séquence d'hyroalumination avec du DIBAL (di-isobutyl-aluminium) /hydrolyse. La séquence hydroalumination / hydrolyse est préférée grace à sa plus grande sélectivité.

L'hydroformylation du composé IV à l'étape 3 est avantageusement catalysée par un complexe organométallique du rhodium, de préférence du [Rh]/P(Ph)₃.

Le schéma 1 ci-après illustre un mode de réalisation préféré du procédé décrit ci-dessus.

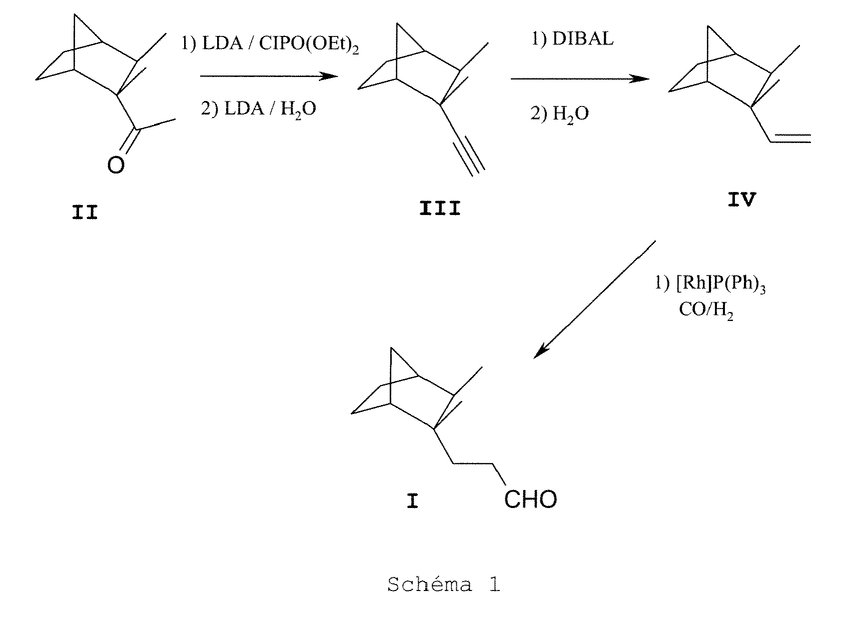

Dans un autre aspect le procédé de l'invention comprend
1. une étape de transformation de la cétone II en le dérivé acétylénique III correspondant, 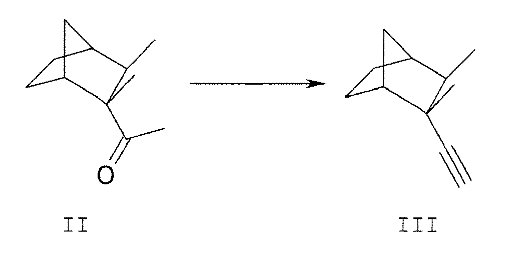
2. une étape de transformation du dérivé III en l'aldéhyde conjugué V correspondant, 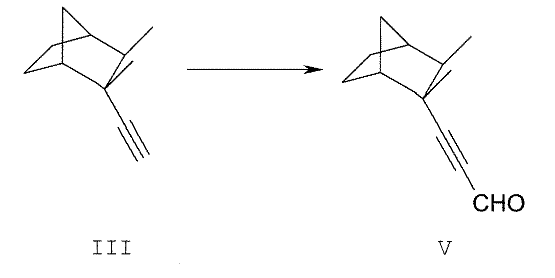
3. une étape de transformation du dérivé V en l'acétal méthylique VI correspondant, et 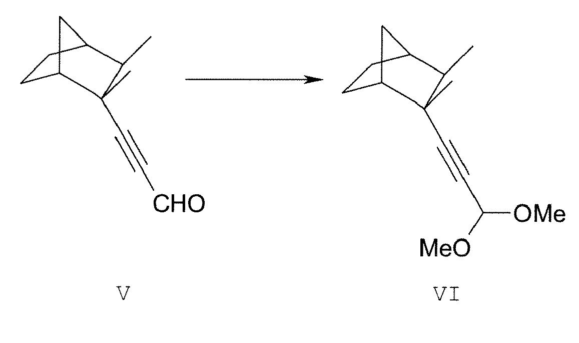
4. une étape d'hydrogénation / hydrolyse acide du dérivé VI pour obtenir l'aldéhyde I.

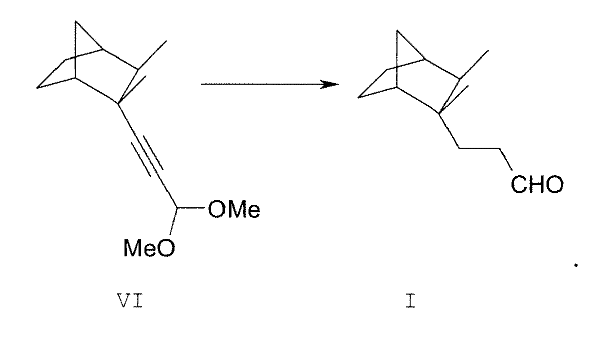

De préférence, la transformation de l'étape 1 est effectuée par une réaction de la cétone II avec 1) du lithium diisopropylamide / diéthylchlorophosphate et 2) du lithium diisopropylamide / H₂O selon Negishi et al, Organic Synthesis, Coll. Vol. VII, 63.

La seconde étape peut être effectuée par une séquence métallation / formylation. De préférence, on utilise du n-BuLi pour la métallation et du diméthylformamide (DMF) pour la formylation.

L'acétal VI de l'étape 3 est avantageusement obtenu par réaction du composé V avec du méthanol sous catalyse acide.

L'hydrogénation de l'acétal VI à l'étape 4 est de préférence catalysée par du charbon palladié.

Le schéma 2 ci-après illustre un mode de réalisation préféré du procédé décrit ci-dessus.

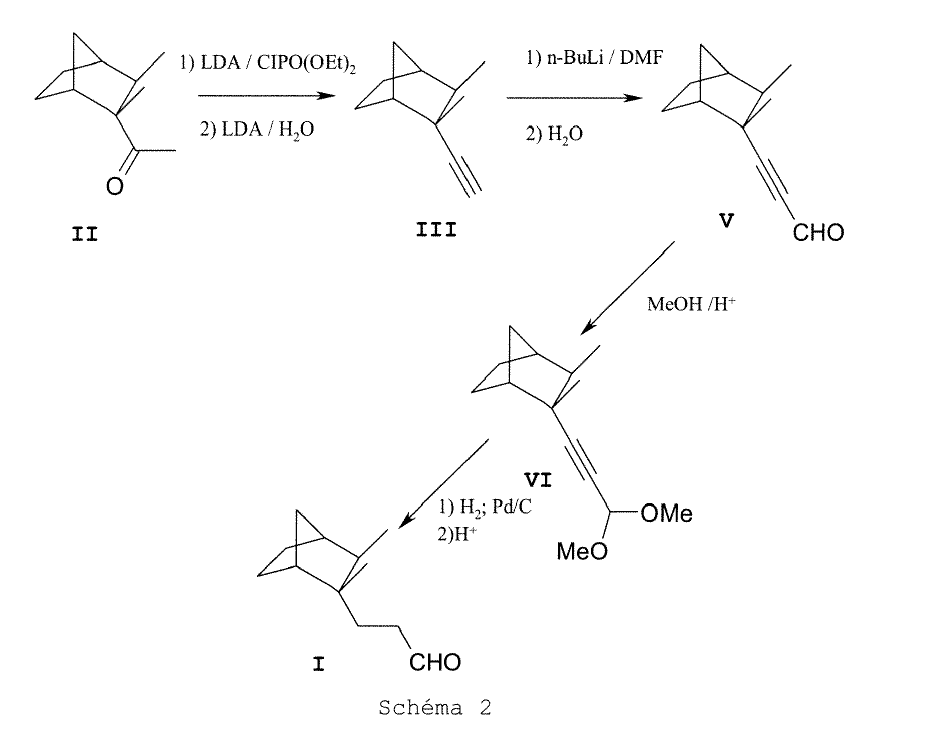

Tout comme l'aldéhyde I, la cétone II existe sous forme de régioisomères II-*endo* et II-*exo*. L'utilisation de l'un des régioisomères II-*endo* ou II-*exo* pur en tant que produit de départ permet la synthèse sélective de l'aldéhyde I-*endo* ou I-*exo.* Ainsi, on obtient l'aldéhyde I-*endo* à partir de la cétone II-*endo* et l'aldéhyde I-*exo* à partir de la cétone II-*exo*.

Ainsi, le procédé selon l'invention peut comprendre, avant la première étape de transformation de la cétone II, une étape de séparation des régioisomères II-*exo* et II-*endo* par distillation.

Lorsque la cétone II est utilisée sous forme de mélange des régioisomères II-*exo* et II-*endo*, l'aldéhyde I est obtenu sous forme d'un mélange I-*exo* et I-*endo* dans les mêmes proportions. Ainsi, l'utilisation de la cétone II sous forme d'un mélange *endo : exo* de 70 : 30 résulte en l'aldéhyde I sous forme d'un mélange *endo : exo* de 70 :30.

La simplicité de mise en oeuvre des réactions et le faible coût des matières premières utilisées, représentent des avantages importants pour la mise en oeuvre industrielle de l'invention.

Les exemples suivants illustrent davantage les différents procédés de fabrication des composés nouveaux selon l'invention ainsi que leur utilisation, et leur intérêt. Ces exemples ne sont présentés que dans un but d'illustration et ne peuvent être considérés comme limitatifs de l'invention.

### Exemple 1 : Synthèse de l'acétylène III

### a) Synthèse du composé III-endo

Dans un ballon tricol de 4 L, muni d'une ampoule à addition, d'un thermomètre et d'un réfrigérant, on place sous azote 90 mL de di*iso*-propylamine (0,640 mole) et 500 mL de THF. La solution est refroidie avec un bain d'acétone /carboglace vers -20 °C. On ajoute alors goutte à goutte, pendant environ deux heures, 300 mL d'une solution de n-butyllithium dans l'hexane dosée à 2,12 M (0,636 mole). A la fin de l'addition, on agite une heure à température ambiante. Puis, on refroidit de nouveau vers -10 °C et on ajoute 100,44 g (0,584 mole) de Présantone H^{®} II-*endo* goutte à goutte pendant une heure. La solution est agitée sous azote à 40 °C pendant vingt heures. Alors, elle est refroidie à -20 °C (bain d'acétone / carboglace) et on y ajoute 100 mL (0,690 mole) de diéthylchlorophosphate goutte à goutte. La solution se colore en orange. On laisse ensuite revenir à température ambiante, on agite trois heures supplémentaires avant d'ajouter goutte à goutte à -78 °C (bain d'acétone saturée en carboglace) 2,2 équivalents par rapport à II-*endo* de LDA, préparés en parallèle de la même façon que précédemment avec 180 mL de di*iso*-propylamine (1,280 moles) et 600 mL d'une solution de n-butyllithium dans l'hexane dosée à 2,12 M (1,272 moles). A la fin de l'addition, la solution est devenue rouge vif. On laisse revenir à température ambiante et on agite une vingtaine d'heures. Puis, la solution est versée sur 500 mL d'un mélange d'acide chlorhydrique à 20% dans l'eau et de glace (50 : 50). Les phases sont séparées. La phase aqueuse est extraite trois fois avec 300 mL de *t*-butyl méthyl éther. Les phases organiques réunies sont lavées par 500 mL d'une solution aqueuse à 10% d'acide chlorhydrique, puis à l'eau, puis avec 300 mL d'une solution aqueuse saturée en bicarbonate de sodium puis deux fois avec 300 mL d'eau. La phase organique est ensuite séchée sur sulfate de magnésium, filtrée, puis concentrée à l'aide de l'évaporateur rotatif. Le produit brut contient 87% de l'acétylénique III-*endo*. Une distillation sous 6 torr à 52 °C permet d'obtenir le 2-éthynyl-2,3-diméthylbicyclo[2.2.1]heptane, III-*endo*, avec un rendement de 65%. 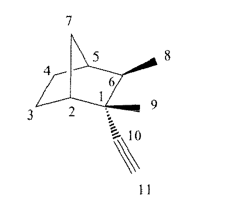
**RMN ¹H : CDCl3, 200MHz :** 2,1 (s, H₁₁) ; 2,0 (m, H₆) ; 1,9 (m, H₂) ; 1,8 (s, H₅) ; 1,8-1,7 (m, H₇) ; 1,6-1,3 (m, H₃ et H₄) ; 1,1 (s, 3H₉) ; 0,9 (d, *J*d=7,4 Hz, 3H₈).
**RMN ¹³C :CDCl3, 50MHz :** 93,3 (C₁₀) ; 67,8 (C₁₁) ; 49,2 (C₂) ; 49,0 (C₅) ; 45,2 (C₆) ; 40,5 (C₁) ; 34,1 (C₇) ; 29,6 (C₄) ; 25,7 (C₃) ; 23,1 (C₉) ; 15,8 (C₈).
**SM :** 148(2) [M^{+.}], 133(52), 120(29), 119(45), 107(13), 106(29), 105(82), 95(18), 93(18), 92(22), 91(100), 80(17), 79(58), 78(13), 77(52), 67(39), 65(21), 55(14), 53(25), 52(12), 51(24), 50(10), 39(45) uma
**IR:** □..cm⁻¹) : 3309, 2105), 1477

### a) Synthèse de III-exo

De la même façon, à partir de 100,12 g (0,603 mole) de Présantone H^{®} II-*exo* avec 864 mL (2,030 mole) de BuLi dosé à 2,35 M dans l'hexane, 290 mL (2,064 moles) de diiso-propylamine et 116 g (0,830 mole) de diéthylchlorophosphate, dans 1 L de THF, on obtient, après distillation (50 °C sous 6 torr), le 2-éthynyl-2,3-diméthylbicyclo[2.2.1]heptane III-*exo*, avec un rendement de 66%. 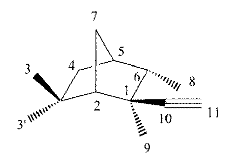
**RMN ¹H : CDCl3, 200MHz :** 2,3-2,1 (m, H₆, H₂) ; 2,1 (s, H₁₁) ; 2,1 (s, H₅) ; 1,9 (m, H₃) ; 1,5-1,2 (m, 2H₄, H₃, et 2H₇) ; 1,1 (s, 3H₉) ; 0,9 (d, *J*d=7,4 Hz, 3H₈).
**RMN ¹³C :CDCl3, 50MHz :** 96,2 (C₁₀) ; 66,7 (C₁₁) ; 50,2 (C₂) ; 45,4 (C₅) ; 43,3 (C₇) ; 38,7 (C₆) ; 37,3 (C₁) ; 23,6 (C₃) ; 20, 6 (C₄) ; 19, 7 (C₉) ; 11,8 (C₈).
**SM :** 148(2) [M^{+.}], 133(50), 120(22), 119(42), 107(13), 106(31), 105(82), 95(15), 93(16), 92(22), 91(100), 80(14), 79(50), 78(13), 77(48), 67(37), 65(21), 55(10), 53(25), 51(20), 39(45) uma
**IR :** □.(cm⁻¹) :3309 , 2105 ,1473

### c) Synthèse de III-endo/exo (70:30)

Le composé III (*endo:exo* = 70:30) a été préparé suivant le même protocole que les deux cas précédents à partir de mélange *endo:exo* de 70:30 de II.

### Exemple 2 : Synthèse de l'oléfine IV

### a) Synthèse de IV-endo

Dans un ballon tricol de 500 mL, muni d'une ampoule à addition, d'un thermomètre et d'un réfrigérant, sous d'azote, on place 13,78 g (0,086 mole) de l'acétylénique III-*endo* dans 100 mL de toluène. On refroidit vers 0 °C avant d'ajouter goutte à goutte pendant environ trente minutes, 100 mL d'une solution de Dibal à 1,0 M dans le toluène (0,10 mole). Puis, on poursuit l'agitation à 55 °C pendant une vingtaine d'heures. Puis, à 0 °C, l'excès de Dibal est neutralisé en ajoutant lentement de l'acétate d'éthyle (environ 10 mL). La solution est ensuite versée sur 100 mL d'un mélange d'une solution d'acide chlorhydrique à 10% dans l'eau et de glace (50 : 50). Les phases sont séparées. La phase aqueuse est extraite deux fois avec 50 mL de toluène. Les phases organiques réunies sont lavées par 50 mL d'une solution aqueuse saturée de bicarbonate de sodium, puis deux fois par 50 mL d'eau. La phase organique est ensuite séchée sur sulfate de magnésium, filtrée, puis concentrée à l'évaporateur rotatif. Après distillation sous vide (Eb. = 50 °C / 6 torr), le rendement en oléfine IV-endo, 2,3-diméthyl-2-vinylbicyclo[2.2.1]heptane est de 87% sous forme d'une huile incolore. 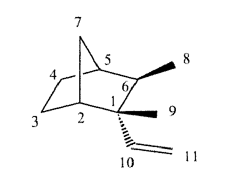
**RMN ¹H : CDCl3, 200MHz :** 6,0-5,9 (m, H₁₀) ; 5,0-4,9 (2dd, *J*₂d=1,6 Hz et *J*₃d=4,4 Hz, *J*₂d=1,4 Hz et *J*₃d=2,8 Hz, 2H₁₁) ; 1,8-1,7 (m, H₆, H₂, H₅) ; 1,6-1,1 (m, 2H₇, 2H₃ et 2H₄) ; 0,9 (s, 3H₉) ; 0,9-0,8 (d, *J*d=7,2 Hz, 3H₈) .
**RMN ¹³C :CDCl3, 50MHz :** 149,0 (C₁₀) ; 110,8 (C₁₁) ; 49,7 (C₆) ; 47,6 (C₁) ; 46,0 (C₂) ; 44,8 (C₅) ; 35,1 (C₇) ; 30,2 (C₃) ; 25,1 (C₄) ; 22,7 (C₉) ; 16,7 (C₈).

### b) Synthèse de composés IV exo et IV (endo/exo)

Les composés IV-exo et IV (*endo*/*exo*) ont été préparés suivant un protocole identique au cas précédent pour le composé IV-endo, respectivement à partir des composés III-*exo* et III (*endo*/ *exo* = 70:30)

### Exemple 3 : Synthèse de l'aldéhyde I

### a) Synthèse de I-endo

Dans un ballon de 250 mL, sec et purgé à l'argon, sont introduits successivement 0.121 g de bis (méthoxy cyclooctadiène rhodium) (0.025 mmoles), 0.262 g de triphénylphosphine (1 mmole), 15 g de l'oléfine IV-*endo* (0.1 mole) puis 100 mL de toluène anhydre et dégazé. La solution résultante est transférée dans un autoclave sous argon et agitée trente minutes. Le mélange est pressurisé sous 5 bars de syngas (CO /H2; 1:1) puis porté à 100 °C pendant deux heures. La solution est refroidie à 20 °C puis filtrée à travers une couche de gel de silice. Après concentration, l'huile obtenue est distillée sous vide (Eb = 50 °C / 10-1 torr). On obtient 14.4 g de l'aldéhyde I-*endo*, le 3-(2,3-diméthylbicyclo[2.2.1]heptan-2-yl)propanal, soit un rendement de 80%. 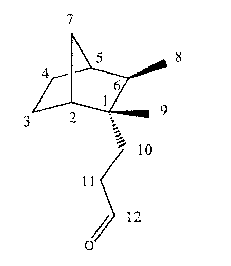
**RMN ¹H : CDCl3, 200MHz :** 9,6 (t, *J*t=1,9 Hz, H₁₂) ; 2,2-2,1 (td, *J*t=6,9 Hz, *J*d=1,9 Hz, 2H₁₁) ; 1,6-1,5 (m, H₆, H₂, H₅) ; 1,4-1,1 (m, 2H₁₀, H_{7'}, H₃ et H₄) ; 1,0-0,8 (m, H_{3'}, H₇, H_{4'}) ; 0,7-0,6 (d, *J*d=6,6 Hz, 3H₈) ; 0,6 (s, 3H₉).
**RMN ¹³C :CDCl3, 50MHz :** 203,5 (C₁₂) ; 48,5 (C₂) ; 47,8 (C₅) 45,8 (C₆) ; 43,0 (C₁) ; 41,0 (C₁₁) ; 35,5 (C₇) 33,1 (C₁₀) ; 30,1 (C₄) ; 24,2 (C₃) ; 21,2 (C₉) ; 17,1 (C₈).
**SM :** 180(1) [M^{+.}] , 133(11), 123(25), 122(46), 121(23), 107(41), 105(10), 97(19), 96(12), 95(87), 94(35), 93(45),91(28),85(15), 84(10), 83(19), 82(18), 81(60),80(10),79(46),77(24),70(17),69(39), 68(26), 67(96), 66(15), 65(17), 57(12), 56(10), 55(100), 53(33), 43(30), 42(10), 41(89), 39(40) uma
**IR :** σ (cm⁻¹) = 2740, 1732, 1479.

### b) Synthèse de l'aldéhyde I-exo et des aldéhydes I (endo/exo)

Les aldéhydes I-*exo* et I (*endo*/*exo)* ont été préparés par une voie identique à celle décrite pour l'aldéhyde I-*endo*, respectivement à partir de III-*exo* et III (*endo*/ *exo* = 70:30)

Données spectrales pour l'aldéhyde I*-exo,* 3-(2,3-diméthylbicyclo[2.2.1]heptan-2-yl)propanal : 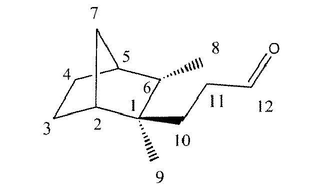
**RMN ¹H : CDCl3, 200MHz :** 9,6 (s, H₁₂) ; 2,2-2,1 (m, 2H₁₁) ; 1,8 (s, H₂) ; 1,6 (s, H₅) ; 1,4-1,2 (m, H₆, 2H₁₀, H₃ et H₄) ; 1,1-0,9 (m, H_{3'}, H_{4'}, 2H₇) ; 0,6 (d, *J*d=7,2 Hz, 3H₈) ; 0,5 (s, 3H₉) .
**RMN ¹³C :CDCl3, 50MHz** :203,7 (C₁₂) ; 47,6 (C₂) ; 44,1 (C₅) ; 43,9 (C₇) ; 40,0 (C₆) ; 39, 6 (C₁) ; 37,4 (C₁₁) ; 36,1 (C₁₀) ; 25,1 (C₃) ; 20,5 (C₄) ; 18,2 (C₉) ; 12,3 (C₈).
**SM :** 180(1) [M^{+.}], 133(10), 123(54), 122(37), 121(20), 107(40), 105(10), 97(17), 96(11), 95(79), 94(29), 93(42), 91(27), 85(14), 82(18), 81(74), 79(46), 77(23), 70(12), 69(36), 68(25), 67(100), 66(13), 65(17), 57(11), 55(94), 53(31), 43(27), 41(83), 39(37) uma.
**IR :** σ (cm⁻¹) = 2738 (w, CHO), 1730 (s, CHO), 1475 (w, CH₃).

### Exemple 4 : Synthèse de l'aldéhyde V

### a) Synthèse de l'aldéhyde V-endo

Dans un ballon tricol de 1 L, muni d'une ampoule à addition, d'un thermomètre et d'un réfrigérant, sous azote, on place 55,07g (0,364 mole) de l'acétylénique III-endo dans 200 mL de THF. On refroidit vers -20 °C avant d'ajouter au goutte à goutte pendant environ une heure, 175 mL d'une solution de n-butyllithium dans l'hexane, dosée à 2,35M. Puis, on poursuit l'agitation à température ambiante pendant une heure, avant d'ajouter par un goutte à goutte lent, à -78 °C, 86 mL (1,1 mole) de diméthylformamide (DMF). On agite quatre heures à -78 °C. La solution est ensuite versée rapidement sur 100 mL d'une solution d'acide chlorhydrique à 10% dans l'eau et de glace (50:50). Les phases sont séparées. La phase aqueuse est extraite cinq fois avec 50 mL de dichlorométhane. Les phases organiques réunies sont lavées par 50 mL d'une solution aqueuse saturée de bicarbonate de sodium, puis deux fois par 50 mL d'eau. La phase organique est ensuite séchée sur sulfate de magnésium, filtrée, puis concentrée à l'évaporateur rotatif.

Le produit brut, contient 93% de l'aldéhyde acétylénique III-*endo* souhaité ainsi que 7% d'acétylénique de départ III-endo. Ils sont séparés par distillation : III-endo bout à 52 °C sous 6 torr et V-endo à 68 °C sous 2.10-1 torr. On obtient 52,3 g de V-endo, le (2,3-diméthylbicyclo[2.2.1]hept-2-yl)-propynal, avec un rendement de 79%. 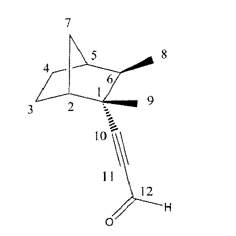
**RMN ¹H** : **CDCl3, 200MHz :** 9,2 (s, N₁₂) ; 2,0 (s, H₂) ; 1,9 (m, H₆, H₅, H_{7'}) ; 1,7-1,6 (m, H₃ et H₄) ; 1,5-1,3 (m, H_{3'}, H₇, H_{4'}) ; 1,2 (s, 3H₉) ; 0,9 (d, *J*d=7,4 Hz, 3H₈).
**RMN ¹³C :CDC13, 50MHz :** 177,9 (C₁₂) ; 108,0 (C₁₀) ; 82,4 (C₁₁) ; 49,4 (C₂) ; 49,4 (C₅) ; 45,4 (C₆) ; 41,6 (C₁) ; 34,6 (C₇) ; 29,8 (C₄) ; 26,2 (C₃) ; 22,4 (C₉) ; 16,1(C₈).
**SM :** 176(1) [M^{+.}], 161(52), 147(77), 143(13), 134(11), 133(65), 120(16), 119(47), 117(17), 115(12), 109(22), 108(17), 107(19), 106(15), 105(66), 103(11), 95(31), 94(16), 93(22), 92 (16), 91(100), 82(11), 81(11), 80(13), 79(81), 78(19), 77(77), 69(12), 68(13), 67(65), 66(14), 65(38), 63(17), 55(32), 53(49), 52(17), 51(42), 50(15), 43(12), 41(83), 40(12), 39(72) uma
**IR :** σ (cm⁻¹) = 2738, 2198 1666, 1468.

### b) Synthèse de l'aldéhyde V-exo

Le (2,3-diméthylbicyclo[2.2.1]hept-2-yl)-propynal V-*exo* est préparé dans les mêmes conditions : A partir de 55,16 g (0,365 mole) de l'acétylénique III-*exo*, en utilisant 175 mL (0,411 mole) de n-BuLi (dosé à 2,35 M dans l'hexane) et 86 mL (1,111 moles) de DMF, on obtient, après distillation (66 °C sous 1,5.10⁻¹ torr), le (2,3-diméthylbicyclo[2.2.1]hept-2-yl)propynal V-*exo*, avec un rendement de 77%. 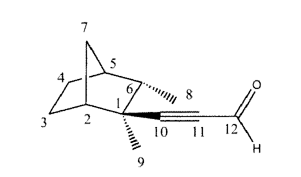
**RMN ¹H : CDCl3, 200MHz :** 9, 2 (s, H₁₂) ; 2,9 (s large, H₆, H₂, H₅) ; 2,2-2,0 (m, H₃ et H₄) ; 1,4-1,0 (m, H_{3'}, H₇, H_{4'}) ; 1,1 (s, 3H₉) ; 0,9 (d, *J*d=7,0 Hz, 3H₈.
**RMN ¹³C :CDCl3, 50MHz :** 178,2 (C₁₂) ; 109,8 (C₁₀) ; 92,0 (C₁₁) ; 60,9 (C₁) ; 49,7(C₂) ; 45,0 (C₅) ; 38,9 (C₇) ; 38,8 (C₆) ; 23,5 (C₃) ; 20,5 (C₄) ; 15,4 (C₉) ; 11,7 (C₈).
**SM :** 176(1) [M^{+.}], 161(54), 148(17), 147(84), 143(12), 133(55), 120(14), 119(48), 117(17), 115(11), 109(36), 108(17), 107(20), 106(13), 105(64), 103(12), 95(41), 94(20), 93(24), 92(15), 91(100), 82(13), 81(13), 80(15), 79(91), 78(20), 77(88), 69(13), 68(18), 67(76), 66(17), 65(41), 63(16), 55(31), 53(48), 52(16), 51(39), 50(14), 43(14), 41(85), 40(11), 39(70).
**IR :** σ (cm⁻¹) = 2738, 2194, 1666, 1465.

### c) Synthèse de l'aldéhyde V-endo/exo

L'aldéhyde V *(endo*/*exo)* a été préparé dans les mêmes conditions que les cas précédents, à partir d'un mélange 70:30 de III avec un rendement de 82%.

### Exemple 5 : Synthèse de l'acétal VI

### a) Synthèse de l'acétal VI-endo

Dans un ballon tricol de 1 L, muni d'une ampoule à addition, d'un thermomètre et d'un réfrigérant, on place 44,43 g (0,235 mole) d'aldéhyde acétylénique V-*endo* dans 250 mL de méthanol. On ajoute 130 mL (1,2 moles) d'orthoformiate de triméthyle et 0,45 g (0,002 mole) d'acide *p*-toluénsulfonique. On agite 24 heures à température ambiante. La solution est ensuite versée sur 100 mL d'une solution aqueuse saturée de bicarbonate de sodium. Les phases sont séparées. La phase aqueuse est extraite trois fois avec 80 mL d'hexane. Les phases organiques réunies sont lavées deux fois par 50 mL d'eau. La phase organique est ensuite séchée sur sulfate de magnésium, filtrée, puis concentrée à l'évaporateur rotatif.

Le produit brut contient 98% de l'acétal acétylénique VI-*endo.* Il sera utilisé pour la prochaine étape sans purification. Le rendement en 2-(3,3-diméthoxyprop-1-ynyl)-2,3-diméthylbicyclo[2.2.1]heptane ,VI-*endo* est quantitatif. 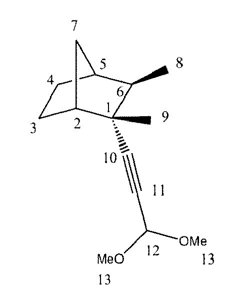
**IR :** σ(cm⁻¹) = 2830, 2235, 1466, 1058.

### b) Synthèse de l'acétal VI-exo

Le 2-(3,3-diméthoxyprop-1-ynyl)-2,3-diméthylbicyclo[2.2.1] heptane, acétal VI-*exo* est préparé dans les mêmes conditions: A partir de 25,01 g (0,142 mole) de l'acétylénique V-*exo*, en utilisant 80 mL (0,731 mole) d'orthoformiate de triméthyle, 1 g (∼4 mol%) d'acide p-toluènsulfonique, dans 200 mL de méthanol, on obtient le 2-(3,3-diméthoxyprop-1-ynyl)-2,3-diméthylbicyclo[2.2.1]heptane VI-*exo* avec un rendement quantitatif.

### c) Synthèse de l'acétal VI (endo/exo)

Le mélange V (*endo*/*exo*) est transformé dans les mêmes conditions que V-*exo* ou V-endo en VI (*endo*/*exo*) dans un rendement de 94%.

### Exemple 6 : Synthèse de l'aldéhyde I (2ème méthode)

### a) Synthèse de l'aldéhyde I-endo

Dans un réacteur d'autoclave, on place 1,0 g de palladium sur charbon (5%), 100 mL d'hexane distillé puis 20,8 g (0,092 mole) d'acétal acétylénique VI-*endo*. On purge trois fois le réacteur à l'azote puis on le place sous 20 bars d'hydrogène. Après une vingtaine d'heures d'agitation, le réacteur est purgé trois fois à l'azote puis la solution est filtrée sur terre d'infusoire. Celle-ci est rincée à l'hexane. Le filtrat est évaporé sous vide.

Le produit brut contient 98% de l'acétal saturé VII-endo. Il sera utilisé pour la prochaine étape sans purification (mais il peut être distillé à 65 °C sous 1,4.10⁻¹ torr). Le rendement en 2-(3,3-diméthoxypropyl)-2,3-diméthylbicyclo [2.2.1]heptane, VII-*endo* est de 93%.

Dans un ballon monocol de 500 mL on place 19,62 g (0,085 mole) d'acétal saturé précédent dans 100 mL de THF. On ajoute 150 mL d'une solution aqueuse d'acide chlorhydrique à 1%. On agite une vingtaine d'heures à température ambiante, puis on ajoute 50 mL d'une solution aqueuse saturée de bicarbonate de sodium. On agite quelques minutes avant d'extraire trois fois avec 100 mL de *t*-butyl méthyl éther. Les phases organiques réunies sont lavées avec 100 mL d'eau. La phase organique est ensuite séchée sur sulfate de magnésium, filtrée, puis concentrée à l'évaporateur rotatif.

Le produit brut contient 94% de l'aldéhyde saturé I-endo. Il est distillé à 52 °C sous 2.10⁻¹ torr. On obtient 13,05 g de 3-(2,3-diméthylbicyclo[2.2.1]heptan-2-yl)propanal, I-*endo* avec un rendement de 83%.

Le produit est identique à celui obtenu précédemment (exemple 3).

### b) Synthèse de l'aldéhyde I-exo

On peut également, dans les mêmes conditions, préparer le 2-(3,3-diméthoxypropyl)-2,3-diméthylbicyclo[2.2.1]heptane VI-*exo.* A partir de 21,0 g (0,095 mole) de l'acétal acétylénique V-*exo* dans l'hexane sous 60 bars d'hydrogène, on obtient, après distillation (62°C/1,4.10⁻¹ torr), le 2-(3,3-diméthoxypropyl)-2,3-diméthylbicyclo[2.2.1]heptane, VI-*exo*, avec un rendement de 93%.

A partir de 10,50 g (0,046 mole) de VI-*exo*, en utilisant 100 mL d'une solution aqueuse d'acide chlorhydrique à 1%, dans 100 mL de THF, on obtient, après distillation (47 °C/1,9.10⁻² torr), le 3-(2,3-diméthylbicyclo[2.2.1]heptan-2-yl)propanal, I-*exo* avec un rendement de 71%.

Le produit est identique au produit obtenu précédemment (exemple 3).

### Exemple 7 : évaluation olfactive

L'impact et les caractéristiques olfactives du composé I-*endo* ont été évalués lorsqu'il est appliqué sur un support. Une base shampoing standard a été choisie. Une composition parfumante a été préparée en mélangeant les ingrédients listés ci-après. Les essais ont été menés en ajoutant 50 parties en poids de composé I-*endo* au mélange de base.

| **COMPOSANTS** | **ESSAI 1** | **ESSAI 2** |
|---|---|---|
| STYRAX RECO VMF | 5 | 5 |
| ISOBORNYLCYCLOHEXANOL à 50% EDG | 90 | 90 |
| YLANG RECO ¹⁾ | 25 | 25 |
| BENZYLE ACETATE | 80 | 80 |
| PHENYLETHYLIQUE ALCOOL | 80 | 80 |
| ANISIQUE ALDEHYDE | 10 | 10 |
| BACDANOL ²⁾ | 20 | 20 |
| CITRONELLOL | 55 | 55 |
| COUMARINE | 35 | 35 |
| DIHYDROMYRCENOL | 20 | 20 |
| ETHYL VANILLINE | 7 | 7 |
| GALAXOLIDE ³⁾ | 140 | 140 |
| GERANIOL | 80 | 80 |
| HELIOTROPINE | 10 | 10 |
| IRISONE PURE ⁴⁾ | 25 | 25 |
| LILIAL | 45 | 45 |
| METHYLIONONE | 25 | 25 |
| BENZYLE SALICYLATE | 80 | 80 |
| VANILLINE | 8 | 8 |
| VERTENEX ⁵⁾ | 80 | 80 |
| ALDEHYDE C10 CAPRIQUE à 10% DPG | 10 | 10 |
| ALDEHYDE C12 LAURIQUE à 10% DPG | 15 | 15 |
| INDOLE à 10% DPG | 5 | 5 |
| COMPOSE **I-*endo*** | | 50 |
| DPG | 50 | |

| | | |
|---|---|---|
| 1) V.MANE FILS 2) 4-(2, 2, 3-triméthyl-3-cyclopentényl)-2-éthyl-3-butèn-1-ol - origine IFF (International Flavors & Fragrance Inc.) 3) 1,3,4,6,7,8- hexahydro-4,6,6,7,8,8-hexaméthylcyclopenta-(γ)-2-benzan-origine IFF (International Flavors & Fragrance Inc.) 4) Mélange d'ionones 5) 4-tert-butylcyclohexyl acetate - origine IFF (International Flavors & Fragrance Inc.) | | |

On observe, par comparaison des essais 1 et 2, que le composé I-*endo* apporte une note de tête et une couverture de base intéressantes avec un caractère boisé-ambré avec des notes patchouli et iris, riches et originales. Ces résultats montrent sans le moindre doute que le composé I-*endo* présente des caractéristiques olfactives intéressantes, susceptibles d'applications dans le domaine de la parfumerie.

## Revendications

1. Composé de formule I :

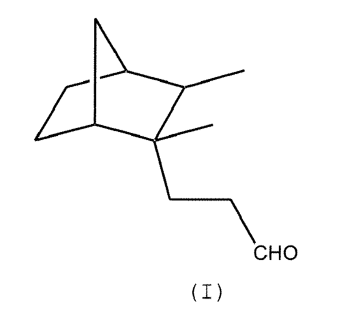

2. Composé selon la revendication 1 sous la forme du régioisomère I-*endo* :

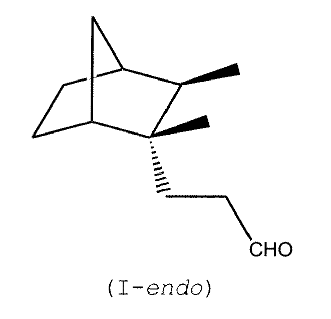

3. Composé selon la revendication 1 sous la forme du régioisomère I-*exo* :

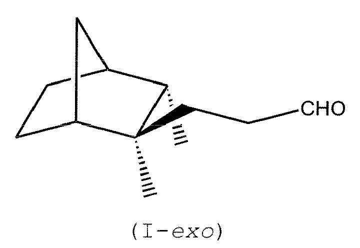

4. Composé selon la revendication 2 ou 3 sous forme d'un énantiomère.

5. Composé selon la revendication 2 ou 3 sous forme d'un mélange d'énantiomères.

6. Composé selon la revendication 5 sous forme d'un racémique.

7. Procédé de fabrication d'un composé selon l'une quelconque des revendications 1 à 5, **caractérisé en ce que** le produit de départ ou un intermédiaire est le composé II de formule :

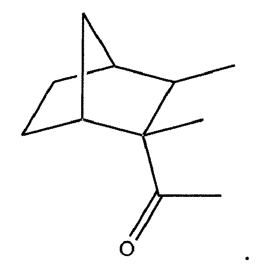

8. Procédé de fabrication selon la revendication 7, comprenant :
1. une étape de transformation de la cétone II en le dérivé acétylénique III correspondant, 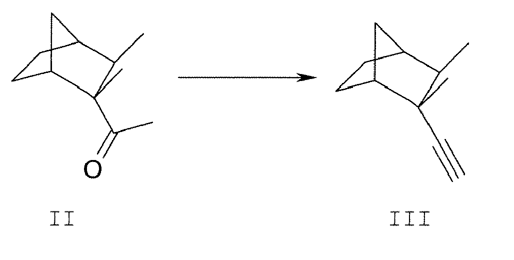
2. une étape transformation du dérivé III en l'oléfine IV correspondante, et 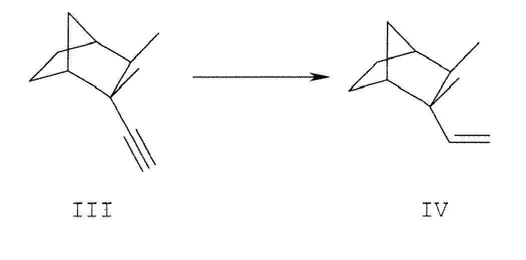
3. une étape d'hydroformylation du dérivé IV pour obtenir l'aldéhyde I.

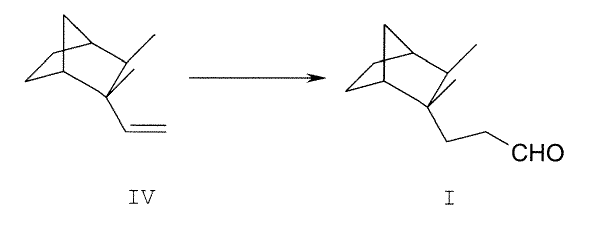

9. Procédé de fabrication selon la revendication 7, comprenant :
1. une étape de transformation de la cétone II en le dérivé acétylénique III correspondant, 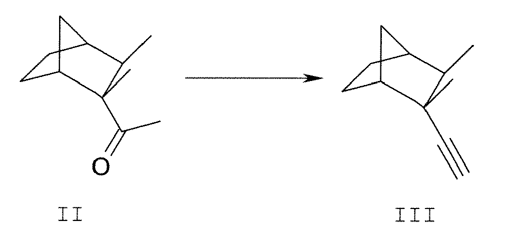
2. une étape de transformation du dérivé III en l'aldéhyde conjugué V correspondant, 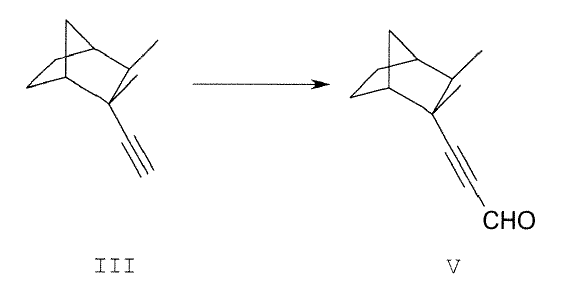
3. une étape de transformation du dérivé V en l'acétal méthylique VI correspondant, et 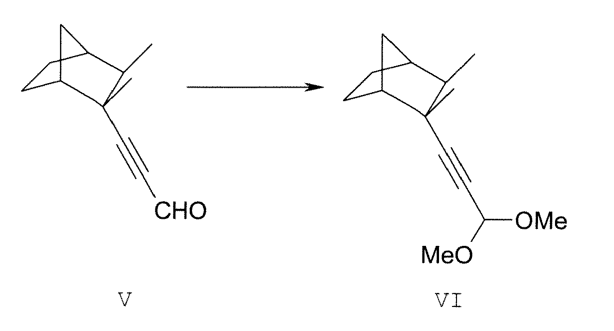
4. une étape d'hydrogénation / hydrolyse acide du dérivé VI pour obtenir l'aldéhyde I

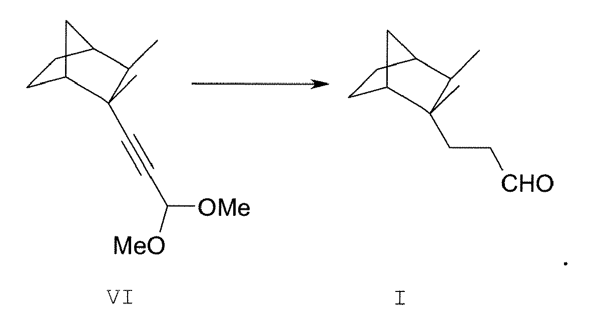

10. Utilisation d'un composé selon l'une quelconque des revendications 1 à 6 en tant qu'agent ou composé odorant, notamment en tant que fragrance ou arôme.

11. Utilisation d'un composé selon l'une quelconque des revendications 1 à 6 pour la préparation de compositions parfumantes, de bases ou concentrés parfumants, parfums ou eaux de toilette, savons, bain moussant, gels douches ou de bain, lotions après rasage, préparations cosmétiques, désodorisants corporels ou d'air ambiant, ou encore détergents ou adoucissants textiles, produits d'entretien et d'assainissement de l'air ambiant.

12. Utilisation d'un composé selon l'une quelconque des revendications 1 à 6 en tant qu'agent masquant d'une odeur.

13. Utilisation selon l'une quelconque des revendications 10 à 12, dans laquelle le composé selon l'une quelconque des revendications 1 à 6 est en combinaison avec au moins un autre ingrédient aromatisant ou parfumant, et/ou au moins un solvant, et/ou au moins un adjuvant.

14. Utilisation selon l'une quelconque des revendications 10 à 12, dans laquelle le composé selon l'une quelconque des revendications 1 à 6 est présent dans une concentration égale à 0,001% à 99% en poids, de préférence de 0,001% à 20% en poids, très préférentiellement 0,1% à 10% en poids, par rapport au poids total.

15. Produit parfumé, notamment parfum et eau de toilette, cosmétique tels que savon, gel douche ou de bain, shampoing et autre produit d'hygiène capillaire, produit d'entretien, lessive assouplissant, désodorisant corporel ou d'air ambiant, **caractérisé en ce qu'**il comprend un composé selon l'une quelconque des revendications 1) à 5).

16. Produit parfumé, produit cosmétique produit d'entretien, lessive, assouplissant textile, désodorisant corporel ou désodorisant d'air ambiant, **caractérisé en ce qu'**il comprend un composé selon l'une quelconque des revendications 1 à 6.
